Europäisches Patentamt

European Patent Office

Office européen des brevets

(19)

(11) Veröffentlichungsnummer: **0 138 040**
**B1**

(12) **EUROPÄISCHE PATENTSCHRIFT**

(45) Veröffentlichungstag der Patentschrift: **31.10.90**

(21) Anmeldenummer: **84110666.9**

(22) Anmeldetag: **07.09.84**

(51) Int. Cl.⁵: **C 12 N 9/06,** C 12 P 7/40, C 12 N 1/16 // C12P41/00 ,(C12N1/16, C12R1:645)

(54) **L-Aminosäure-Oxidase aus Hefen der Gattung Cryptococcus, ihre Herstellung un Verwendung.**

(30) Priorität: **16.09.83 DE 3333453**

(43) Veröffentlichungstag der Anmeldung: **24.04.85 Patentblatt 85/17**

(45) Bekanntmachung des Hinweises auf die Patenterteilung: **31.10.90 Patentblatt 90/44**

(84) Benannte Vertragsstaaten: **AT BE CH DE FR GB IT LI LU NL SE**

(56) Entgegenhaltungen:
FR-A-2 418 239
FR-A-2 522 680
GB-A-2 075 026

CHEMICAL ABSTRACTS, Band 84, Nr. 21, 24. Mai 1976, Seite 435, Zusammenfassung Nr. 149208r, Columbus, Ohio, US; & JP-A-75 135 280 (TANABE SEIYAKU CO. LTD) 27-10-1975

(73) Patentinhaber: **HOECHST AKTIENGESELLSCHAFT**
**Postfach 80 03 20**
**D-6230 Frankfurt am Main 80 (DE)**

(72) Erfinder: **Aretz, Werner, Dr.**
**Am Krummorgen 2**
**D-6233 Kelkheim (Taunus) (DE)**
Erfinder: **Sauber, Klaus, Dr.**
**Falkenstrasse 35**
**D-6232 Bad Soden am Taunus (DE)**

(56) Entgegenhaltungen:

CHEMICAL ABSTRACTS, Band 88, Nr. 1, 1. Februar 1978, Seite 424, Zusammenfassung Nr. 4768w, Columbus, Ohio, US; & JP-A-77 108 086 (AJINOMOTO CO. INC.) 10-09-1977

Courier Press, Leamington Spa, England.

**Beschreibung**

Die Erfindung betrifft eine neue L-Aminosäure-Oxidase mit breitem Substratspektrum, ihre Gewinnung durch Fermentation von Hefen der Gattung Cryptococcus und ihre Verwendung zur Herstellung von α-Ketosäuren, ihren Estern und Ethern aus den entsprechenden L-α-Aminosäuren bzw. deren Derivaten.

"L-Aminosäure-Oxidasen wurden schon als Bestandteil von Schlangengift, in Nieren von Ratten und Mäusen, aber auch in Bakterien und Pilzen gefunden (Arch. Biochem. Biophys. (1971) *146*: 54—63; J. Bacteriol. (1975) *121*: 656—663; FR—8303412; UK 2075026 A; JP—A—75 135 280; FR—A—241 8239; FR—A—2522 680; JP—A—77 108 086)

Die bisher bekannten L-Aminosäure-Oxidasen akzeptieren als Substrat im wesentlichen die proteinogenen Aminosäuren, wie z.B. Lysin, Tyrosin oder Phenylalanin, reagieren aber auch mit Ornithin und Citrullin. Die erfindungsgemäße L-Aminosäure-Oxidase kann neben vielen proteinogenen Aminosäuren auch L-Aminoadipinsäure, L-Cephalosporin C, sowie Ester und Ether von proteinogenen L-Aminosäuren oxidieren."

Die L-Aminosäure-Oxidase, im folgenden LAO, stellt für die Hefen der Gattung Cryptococcus ein induzierbares Enzym dar. Zu ihrer Herstellung fermentiert man deshalb die Hefen unter Zusatz einer Aminosäure oder eines Aminosäuren freisetzenden Stoffs als Induktor. Bevorzugte Ausgestaltungen der Erfindung werden im folgenden näher erläutert:

Bevorzugt aus der Gattung Cryptococcus ist die Art C. laurentii, beispielsweise der Stamm Cryptococcus laurentii var. magnus CBS 569, sowie die Art C. albidus.

Besonders bevorzugt ist der Stamm C. laurentii DSM 2762. Ausgangsmaterial für diesen Stamm war eine Erdprobe aus Bobodiovlassio (Obervolta), die über mehrere Passagen in einem Mineralmedium mit D-Glutaminsäure als einziger Stickstoffquelle bei 28°C für jeweils 2—3 Tage inkubiert wurde. Diese Flüssigkulturen wurden auf Medien ausplattiert, die D-α-Aminoadipinsäure-Ethylamid als einzige N-Quelle enthielten. Nach weiteren Überimpfungen wurde u.a. der Stamm DSM 2762 als Reinkultur isoliert.

Bei diesem Stamm handelt es sich um eine einzellige, ovale Hefe, die weder Mycel noch Pseudomycelien bildet. Die Vermehrung vollzieht sich durch vielseitige Sproßung; ein Vorhandensein von Asko- bzw. Ballistosporen konnte nicht nachgewiesen werden. Die konvexen, weißlichen Kolonien sind rauh und haben einen glatten Rand. Eine Pigmentbildung in Form von Carotinoiden findet nicht statt. Der Nachweis von Hefestärke gelang mit Jod-Jodkalium, und zwar sowohl in den Kolonien als auch in Flüssigkulturen. Physiologische Untersuchungen zeigten, daß Glucose, Saccharose, Maltose, Raffinose, Galaktose, Lactose, Stärke, Rhamnose, Melibiose, Dextrin und Inosit als Kohlenstoffquelle assimiliert werden; eine Vergärung der Zucker findet nicht statt. Eine Verwertung von Ammoniumsulfat, α-Aminoadipinsäure, Glutaminsäure, Alanin, Leucin, Serin, Tryptophan, Tyrosin und Phenylalanin als Stickstoffquelle konnte gezeigt werden. Ein Wachstum mit Natriumnitrat wurde hingegen nicht beobachtet.

Es wurde gefunden, daß LAO parallel zur Wachstum gebildet wird und ihre höchste Aktivität gegen Ende der logarithmischen Phase erreicht. Bevorzugte Induktoren sind D-Aminosäuren, insbesondere D-

Leu, D-α-Aminoadipinsäure (DαAAA) sowie D-Ala. Eine Übersicht über gefundene LAO-Aktivitäten zeigt die Tabelle 1:

TABELLE 1

Induktion der L-Aminosäure-Oxidase Durch Verschiedene Aminosäuren

| N-Quelle | OD 546 nm | LAO-Aktivität U/g Zellen* |
|---|---|---|
| NH$_4$ Cl | 9,5 | 0 |
| D-Ala | 1,9 | 1,22 |
| DL-Ala | 11,8 | 0,93 |
| D-αAAA | 9,3 | 1,50 |
| L-αAAA | 9,0 | 1,25 |
| DL-αAAA | 8,9 | 1,76 |
| D-Leu | 5,0 | 3,70 |
| L-Leu | 6,2 | 1,20 |
| DL-Leu | 4,2 | 1,60 |
| L-Met | 3,1 | 0,70 |
| DL-Phe | 3,6 | 0,74 |
| L-Try | 3,9 | 0,50 |
| L-Ser | 13,3 | 0,30 |
| D-Glu | 10,2 | 0 |
| DL-Glu | 15,7 | 0 |

* Testsubstrat L-α-Aminoadipinsäure (LαAAA)

Bevorzugte C-Quellen sind lösliche Stärke und insbesondere Lactose und Saccharose.

Im Gegensatz zu den bekannten mikrobiellen L-Aminosäure-Oxidasen weist die erfindungsgemäße LAO ein breites Substratspektrum auf: Neben den meisten natürlichen Aminosäuren werden auch andere Aminosäuren wie L-α-Aminoadipinsäure und L-Cephalosporin C in die entsprechenden α-Ketosäuren umgewandelt. Daneben werden jedoch auch Derivate der Aminosäuren umgesetzt, nämlich ihre Ester, insbesondere niedere Alkylester und Benzylester, sowie die Ether und zwar sowohl Ether der Alkoholgruppe des Serins als auch der phenolischen Hydroxygruppe des Tyrosins und Thioether des Cysteins. Auch hierbei sind die niederen Alkyl- und Benzyl-Ether bzw. -thioether bevorzugt. Der natürliche Thioether L-Methionin wird ebenfalls umgesetzt.

Alle Umsetzungen sind streng stereospezifisch: Es werden die L-Formen in die entsprechenden Ketosäuren bzw. Ketosäurederivate umgewandelt. Erfindungsgemäß können somit auch Racemate getrennt werden, wobei die L-Form zum Ketoderivat umgeformt wird, während die D-Form unverändert bleibt.

Die Umsetzung der L-Aminosäuren erfolgt vorteilhaft in einem pH-Bereich von 6,5—8,5, vorteilhaft 7—8, insbesondere 7,5. Geeignete Puffer sind deshalb Kaliumphosphat- und Tris-HCl-Puffer.

Vorteilhafte Umsetzungstemperaturen liegen bei etwa 30 bis 60, vorzugsweise 40 bis 55, insbesondere 50°C.

Die LAO weist für L-α-AAA einen Km- Wert von 0,25 mM und einen Vmax von 2 mM auf.

Die erfindungsgemäße LAO zeichnet sich durch eine hohe Lagerstabilität aus. Sie ist bei 4°C mehrere Tage und bei −18°C mehrere Monate ohne Aktivitätsabfall verwendbar.

Die erfindungsgemäße LAO ist auf der äußeren Cytoplasmamembran lokalisiert. Die Enzymaktivität ist deshalb in nicht-permeabilisierten, intakten Zellen genauso hoch wie in mit Cetyl-trimethylammoniumbromid-behandelten Zellen. Einfrieren und Auftauen der Zellen bewirkt eine Aktivitätssteigerung um 30 bis 40%.

Die erfindungsgemäße LAO kann als Konzentrat aus der Cytoplasmamembran eingesetzt werden. Besonders vorteilhaft ist jedoch die Verwendung in Form fixierter Zellen. Da — wie vorstehend erwähnt — das Enzym auf der äußeren Cytoplasmamembran lokalisiert ist, ist es nicht erforderlich, bei der Fixierung der Zellen nicht-toxische Bedingungen einzuhalten.

Neben den bekannten Vorteilen der Enzymfixierung — höhere Stabilität und verbesserte Handhabbarkeit — entfällt bei der Einbettung der ganzen Zellen die Isolierung und Reinigung des Enzyms.

**EP 0 138 040 B1**

Die Immobilisierung des Enzyms bzw. der Zellen kann in bekannter Weise mit natürlichen oder synthetischen Polymeren erfolgen (Nachr. Chem. Tech. Lab. 29 (1981) 850, deutsche Offenlegungsschriften 2 252 815, 2 343 633, 2 414 128, 2 420 102 und 2 805 607).

In den folgenden Beispielen werden besonders bevorzugte Ausgestaltungen der Erfindung näher erläutert:

Beispiel 1

Die Hefe Cryptococcus albidus wird auf dem folgenden festen Nährboden gehalten:

| | |
|---|---|
| "Nutrient Broth" | 8 g |
| Agar | 15 g |
| dest. Wasser | 1 l |

Das Medium wird auf Reagenzgläser verteilt und 30 min bei 121°C sterilisiert, anschließend abgekühlt, mit der Kultur beimpft und 3—4 Tage bei 25°C inkubiert. Die gewachsene Kultur wird mit 10 ml steriler Kochsalzlösung abgeschwemmt und in ein Kulturmedium der folgenden Zusammensetzung gegeben:

| | |
|---|---|
| Glucose | 10 g |
| D-α-AAA | 0,3 g |
| $KH_2PO_4$ | 0,875 g |
| $K_2HPO_4$ | 0,125 g |
| NaCl | 0,1 g |
| $MgCl_2 \cdot 7H_2O$ | 0,5 g |
| $CaCl_2 \cdot 7H_2O$ | 0,1 g |
| Spurenelementelösung | 1 ml |
| Vitaminlösung | 10 ml |
| dest. $H_2O$ (pH 7,2) | 1 l |

| Spurenelementelösung: | | Vitaminlösung: | |
|---|---|---|---|
| $CoCl_2 \cdot 6H_2O$ | 0,25 g | Biotin | 0,001 g |
| $NiCl_2 \cdot 6H_2O$ | 0,01 g | Vitamin B 12 | 0,005 g |
| $CuCl_2 \cdot 2H_2O$ | 0,01 g | Thiamin·HCl | 0,03 |
| $ZnCl_2$ | 0,1 g | Nicotinsäure | 0,035 |
| $H_3BO_3$ | 0,5 g | p-Aminobenzoesäure | 0,02 |
| $Na_2MoO_4 \cdot 2H_2O$ | 0,3 g | Pyridoxal·HCl | 0,01 |
| $NaSeO_3 \cdot 3H_2O$ | 0,1 g | Ca-Pantothenat | 0,01 |
| $FeSO_4 \cdot 7H_2O$ | 0,2 g | 50% Ethanol | 1 l |
| dest. $H_2O$ | 1 l | | |

(mit HCl auf pH 2—3 eingestellt)

500 ml dieses Mediums werden in 2 l-Erlenmeyerkolben gegeben und 30 min bei 121°C sterilisiert. Die mit 10 ml Inokulum beimpften Kolben werden anschließend bei 28°C und 190 Upm auf einem Rotationsschüttler inkubiert. Nach 72 Stunden wird die gewachsene Kultur abgeerntet, gewaschen und in einem Kaliumphosphatpuffer (pH 7,5, 50 mM) aufgenommen. Die LAO-Aktivität der intakten Zellen wurde

4

mit L-α-AAA als Substrat in einem o-Phenylendiamin-Peroxydase-abhängigen Test mi 1,34 U/g Zellen bestimmt.

## Beispiel 2

Cryptococcus laurentii DSM 2762 wurde gemäß Beispiel 1 in 500 ml Nährlösung angezogen und nach 3 Tagen in einem 12 l-Fermenter mit 9 Liter des gleichen Mediums, welches jedoch D-Leucin statt D-α-AAA enthält, überimpft und bei 28°C, 400 Upm und eine Belüftungsrate von 400 l Luft pro Stunde inkubiert.
Nach 4 Tagen wurde eine LAO-Aktivität von 3,5 U/g Zellen gemessen.

## Beispiel 3

Eine 6 %ige Lösung von χ-Carrageenan® (Marine Colloids, Rockland, Maine, USA) wird bei 75°C gelöst, auf 40°C abgekühlt und mit einer 4 %igen Zellsuspension von Cryptococcus-Zellen in physiologischer Kochsalzlösung im Verhältnis 1:1 vermischt. Diese Suspension wird durch eine Kanüle in ein Fällbad (10 mM CaCl$_2$, 300 mM KCl) gedrückt, so daß Kugeln entstehen. Nach einstündigem Nachrühren wird dreimal mit 0,3 M KCl gewaschen. Die Carrageenan®-Kugeln werden bei 4°C in 0,13 M Kaliumphosphatpuffer (pH 7,5) der 0,02% Natriumazid enthält, aufbewahrt. Die Aktivität der Kugeln beträgt etwa 80 mU/g Katalysatorfeuchtmasse.

## Beispiel 4

10 ml 10 mM L-Phenylalanin, gelöst in 0,1 M Kaliumphosphatpuffer (pH 8,0) werden mit 4 g nach Beispiel 3 fixierten Cryptococcus laurentii DSM 2762-Zellen unter Luftbegasung bei 37°C umgesetzt. Ein Zusatz von 10 µl technischer Katalase (Boehringer, Mannheim) bewirkt die Zerstörung des entstehenden Wasserstoffperoxids und vermeidet eine Beeinträchtigung der Produktqualität. Das Verschwinden des Substrats und die Bildung des Produkts kann durch Dünnschichtchromatographie verfolgt werden. Der Nachweis des Produkts kann durch Besprühen des Dünnschichtchromatogramms mit 2,4-Dinitrophenylhydrazin erfolgen. Ebenso kann die Bildung von Ammoniumionen mit der Nitroprussid-Methode verfolgt werden.
Nach 5 Stunden ist das Ausgangsmaterial quantitativ umgesetzt.
Die in den folgenden Tabellen 2 und 3 aufgeführten Ergebnisse wurden gemäß Beispiel 4 erhalten. Die Substratkonzentration war 4 mM, sofern nichts anderes angegeben ist.

### TABELLE 2
Substratspektrum der LAO aus Cryptococcus Laurentii DSM 2762

| Substrat | LAO-Aktivität in % | Substrat | LAO-Aktivität in % |
|---|---|---|---|
| L-α-AAA | 100 | D-Ala | 0 |
| L-Ala | 72 | D-α-AAA | 0 |
| | | D-Leu | 0 |
| L-Arg | 78 | D-Meth | 0 |
| L-Asn | 68 | D-Phe | 0 |
| L-Asp | >0 | D-Try | 0 |
| L-Cys | >0 | D-Val | 0 |
| L-Glu | 49 | | |
| L-Gly · | 0 | L-CPC | 76 |
| L-Ile | 40 | D-CPC | 0 |
| | | L-Met-Amid | 0 |
| L-Leu | 73 | L-Leu-Amid | 0 |
| | | L-Try-Amid | 0 |
| L-Lys | 34 | | |
| L-Met | 58 | | |
| L-Phe | 72 | | |
| L-Pro | 0 | | |
| L-Ser | 46 | | |
| L-Thr | 0 | | |
| L-Try | 29 | | |
| L-Tyr | 56 | | |
| L-Val | 0 | | |

## EP 0 138 040 B1

### TABELLE 3
Substratspektrum der LAO aus Cryptococcus Laurentii DSM 2762

| Substrat | LAO-Aktivität in % |
|---|---|
| L-α-AAA | 100 |
| L-Ala-OMe | 98 |
| L-Ala-OEt | 40 |
| L-Ala-OtBu | 32 |
| L-Arg-OMe | 64 |
| L-Leu-OMe | 100 |
| L-Lys-Ome | 109 |
| D,L-Met-OMe | 100 |
| L-Met-OEt | 84 |
| L-Phe-OMe | 116 |
| L-Phe-OEt | 84 |
| L-Phe-OtBu | 69 |
| L-Ser-OMe | 50 |
| L-Ser-OBz | 82 |
| L-(S-Bz)-Cys* | 70 |
| L-(S-Bz)-Cys-OMe** | 63 |
| L-Tyr-Me-ether** | 74 |
| D,L-Val-OMe | 0 |
| L-1-Naphthyl-alanin | 63 |
| L-2-Naphthyl-alanin | 100 |

\* 3 mM
\*\* 2 mM
Me = Methyl
Et = Ethyl
tBu = tert.-Butyl
Bz = Benzyl

**Patentansprüche für die Vertragsstaaten: BE CH DE FR GB IT LI LU NL SE**

1. L-Aminosäure-Oxidase aus Hefen der Gattung Cryptococcus, dadurch gekennzeichnet, daß das Enzym ein breites Substratspektrum aufweist, wobei es insbesondere mit L-Aminoadipinsäure und L-Cephalosporin C reagiert.

2. L-Aminosäure-Oxidase nach Anspruch 1 aus Hefen der Art C. laurentii und/oder C. albidus.

3. L-Aminosäure-Oxidase nach Anspruch 1 aus C. laurentii DSM 2762.

4. Verfahren zur Herstellung der L-Aminosäure-Oxidasen nach Anspruch 1 bis 3, dadurch gekennzeichnet, daß man die genannten Hefen unter Zusatz einer Aminosäure oder eines Aminosäuren freisetzenden Stoffs fermentiert.

5. Verfahren nach Anspruch 4, dadurch gekennzeichnet, daß eine D-Aminosäure eingesetzt wird.

6. Verfahren nach Anspruch 5, dadurch gekennzeichnet, daß D-Leu, D-Ala, D-α-Aminoadipinsäure oder D-α-Aminoadipinsäure-δ-semiethylamid eingesetzt werden.

7. Verfahren nach einem oder mehreren der Ansprüche 4 bis 6, dadurch gekennzeichnet, daß als Kohlenstoffquelle Lactose oder Saccharose dienen.

8. Verwendung der L-Aminosäure-Oxidase gemäß Anspruch 1 bis 3 bzw. der nach Anspruch 4 bis 7 erhältlichen, Produkte zur Herstellung von α-Ketosäuren und ihren Estern und Ethern aus den entsprechenden L-α-Aminosäuren bzw. -derivaten.

9. Verwendung gemäß Anspruch 8, wobei die L-Aminosäure-Oxidase als Konzentrat aus der Cytoplasmamembran oder in Form fixierter Zellen eingesetzt wird.

10. Cryptococcus laurentii DSM 2762.

## Patentansprüche für den Vertragsstaat: AT

1. Verfahren zur Herstellung von L-Aminosäure-Oxidasen, dadurch gekennzeichnet, daß Hefen der Gattung Cryptococcus unter Zusatz einer Aminosäure oder eines Aminosäuren freisetzenden Stoffs fermentiert werden.

2. Verfahren nach Anspruch 1, dadurch gekennzeichnet, daß eine D-Aminosäure eingesetzt wird.

3. Verfahren nach Anspruch 1, dadurch gekennzeichnet, daß D-Leu, D-Ala, D-α-Aminoadipinsäure oder D-α-Aminoadipinsäure-δ-semiethylamid eingesetzt werden.

4. Verfahren nach einem oder mehreren der Ansprüche 1 bis 3, dadurch gekennzeichnet, daß als Kohlenstoffquelle Lactose oder Saccharose dienen.

5. Verfahren nach einem oder mehreren der vorhergehenden Ansprüche, dadurch gekennzeichnet, daß Hefen der Art C. laurentii und/oder C. albidus eingesetzt werden.

6. Verfahren nach einem oder mehreren der vorhergehenden Ansprüche, dadurch gekennzeichnet, daß als Hefe C. laurentii DSM 2762 eingesetzt wird.

7. Verwendung der nach Anspruch 1 bis 6 erhältlichen L-Aminosäure-Oxidase zur Herstellung von α-Ketosäuren und ihren Estern und Ethern aus den entsprechenden L-α-Aminosäuren bzw. -derivaten.

8. Verwendung gemäß Anspruch 7, wobei die L-Aminosäure-Oxidase als Konzentrat aus der Cytoplasmamembran oder in Form fixierter Zellen eingesetzt wird.

## Revendications pour les Etats contractants: BE CH DE FR GB IT LI LU NL SE

1. L-aminoacide oxydase provenant de levures appartenant au genre *Cryptococcus*, caractérisée en ce que l'enzyme présente un large spectre de substrats, et réagit en particulier avec l'acide L-aminoadipique et la L-céphalosporine C.

2. L-aminoacide oxydase selon la revendication 1, provenant de levures appartenant à l'espèce *C. laurentii* et/ou *C. albidus*.

3. L-aminoacide oxydase selon la revendication 1, provenant de *C. laurentii* DSM 2762.

4. Procédé pour la production de la L-aminoacide oxydase selon les revendications 1 à 3, caractérisé en ce que l'on cultive par fermentation les levures citées, avec addition d'un aminoacide ou d'une substance libérant des aminoacides.

5. Procédé selon la revendication 4, caractérisé en ce que l'on utilise un D-aminoacide.

6. Procédé selon la revendication 5, caractérisé en ce que l'on utilise la D-Leu, la D-Ala, l'acide D-α-aminoadipique ou le D-α-aminoadipyl-δ-semi-éthylamide.

7. Procédé selon une ou plusieurs des revendications 4 à 6, caractérisé en ce que l'on utilise en tant que source de carbone le lactose ou le saccharose.

8. Utilisation de la L-aminoacide oxydase selon les revendications 1 à 3 ou préparable selon les revendications 4 à 7, produits pour la préparation d'α-cétoacides et de leurs esters et éthers à partir des L-α-aminoacides correspondants ou de leurs dérivés.

9. Utilisation selon la revendication 8, la L-aminoacideoxydase étant utilisée sous forme de concentré obtenu à partir de la membrane cytoplasmique, ou sous forme de cellules fixées.

10. *Cryptococcus laurentii* DSM 2762.

## Revendications pour l'Etat contractant: AT

1. Procédé pour la production de L-aminoacide oxydases, caractérisé en ce que l'on cultive par fermentation des levures appartenant au genre *Cryptococcus*, avec addition d'un aminoacide ou d'une substance libérant des aminoacides.

2. Procédé selon la revendication 1, caractérisé en ce que l'on utilise un D-aminoacide.

3. Procédé selon la revendication 1, caractérisé en ce que l'on utilise la D-Leu, la D-Ala, l'acide D-α-aminoadipique ou le D-α-aminoadipyl-δ-semi-éthylamide.

4. Procédé selon une ou plusieurs des revendications 1 à 3, caractérisé en ce que l'on utilise en tant que source de carbone le lactose ou le saccharose.

5. Procédé selon une ou plusieurs des revendications précédentes, caractérisé en ce que l'on utilise des levures appartenant à l'espèce *C. laurentii* et/ou *C. albidus*.

6. Procédé selon une ou plusieurs des revendications précédentes, caractérisé en ce que l'on utilise en tant que levure *C. laurentii* DSM 2762.

7. Utilisation de la L-aminoacide oxydase préparable selon les revendications 1 à 6, pour la préparation d'α-cétoacides ou de leurs esters et éthers, à partir des L-α-aminoacides correspondants ou de leurs dérivés.

8. Utilisation selon la revendication 7, la L-aminoacide oxydase étant utilisée sous forme de concentré provenant de la membrane cytoplasmique ou sous forme de cellules fixées.

**Claims for the Contracting States: BE CH DE FR GB IT LI LU NL SE**

1. L-Amino acid oxidase from yeasts of the genus Cryptococcus wherein the enzyme has a wide substrate spectrum and reacts, in particular, with L-aminoadipic acid and L-cephalosporin C.

2. L-Amino acid oxidase as claimed in claim 1 from yeasts of the species C. laurentii and/or C. albidus.

3. L-Amino acid oxidase as claimed in claim 1 from C. laurentii DSM 2762.

4. A process for the preparation of the L-amino acid oxidase as claimed in claim 1 to 3, which comprises fermenting the said yeasts with the addition of an amino acid or a substance which releases an amino acid.

5. The process as claimed in claim 4, wherein a D-amino acid is used.

6. The process as claimed in claim 5, wherein D-Leu, D-Ala, D-α-aminoadipic acid or D-α-aminoadipic acid-δ-semiethylamide are used.

7. The process as claimed in one or more of claims 4 to 6, wherein the carbon source used is lactose or sucrose.

8. The use of the L-amino acid oxidase as claimed in claim 1 to 3, or of the products obtainable as claimed in claim 4 to 7, for the preparation of α-keto acids and their esters and ethers from the corresponding L-α-amino acids or derivatives.

9. The use as claimed in claim 8, where the L-amino acid oxidase is used as a concentrate from the cytoplasmic membrane or in the form of immobilized cells.

10. Cryptococcus laurentii DSM 2762.

**Claims for the Contracting State: AT**

1. A process for the preparation of L-amino acid oxidase, which comprises fermenting yeasts of the genus Cryptococcus with the addition of an amino acid or a substance which releases an amino acid.

2. The process as claimed in claim 1, wherein a D-amino acid is used.

3. The process as claimed in claim 1, wherein D-Leu, D-Ala, D-α-aminoadipic acid or D-α-aminoadipic acid-δ-semiethylamide are used.

4. The process as claimed in one or more of claims 1 to 3, wherein the carbon source used is lactose or sucrose.

5. The process as claimed in one or more of the preceding claims, wherein yeasts of the species C. laurentii and/or C. albidus are used.

6. The process as claimed in one or more of the preceding claims, wherein the yeast used is C. laurentii DSM 2762.

7. The use of the L-amino acid oxidase obtainable as claimed in claim 1 to 6, for the preparation of α-keto acids and their esters and ethers from the corresponding L-α-amino acids or derivatives.

8. The use as claimed in claim 7, where the L-amino acid oxidase is used as a concentrate from the cytoplasmic membrane or in the form of immobilized cells.